# EUROPÄISCHE PATENTANMELDUNG

(11) **EP 3 058 946 A1**
(43) Veröffentlichungstag der Anmeldung: **24.08.2016**
(21) Anmeldenummer: 16000031.1
(22) Anmeldetag: 11.01.2016
(51) Int. Cl.: A61K 36/28, A61K 36/752, A61K 36/9068, A61K 36/235, A61K 36/236, A61K 36/53, A61K 36/282, A61K 36/484, A23L 2/00

(54) **ALKOHOLFREIER SCHWEDENBITTER**

(30) Priorität: 09.01.2015 DE 202015000039 U
(71) Anmelder: Braeunig, Elke, 34497 Korbach (DE)
(72) Erfinder: Braeunig, Elke, 34497 Korbach (DE)
(74) Vertreter: Bittner, Bernhard

(57) **Zusammenfassung**

Die vorliegende Erfindung betrifft eine Alkoholarme oder alkoholfreie Zubereitung, umfassend Wasser, Süßungsmittel und mindestens 12, bevorzugt 18, besonders bevorzugt 24 Kräuter oder Kräuterextrakte, wobei die Kräuter oder Kräuterextrakte aus einer Gruppe ausgewählt sind, die Wermut, Eibisch, Angelika, echte Angostura, Anis, Stemansis, Beifußkraut, Orangenschale, Grüntee, Cardamon, Saflor, Gewürznelken, Tausendguldenkraut, Kamille, Zimtrinde, Koriander, Cubebenpfeffer, Attlchwurzel, Zwergholunderwurzel, Fenchel, Galgant-wurzel, Enzian, Alantwurzel. Artischocke, Pfefferfrüchte (schwarz), Iriswurzel, Wat-nußblätter. Süßholz, Süßholzsaft, Melisse, Bitterklee, Muskatblüte, Macis, Muskatnuss, Myrrha, Salbei, Bohnenkraut, Quendel, wilder Thymian, Thymian, Tormentill, Rotwurzel, Zittwerwurzel und Ingwer oder deren Extrakte umfasst. Bevorzugt handelt es sich bei einer solchen Zubereitung um einen Schwedenbitter. Weiterhin betrifft die Erfindung ein Verfahren zu Herstellung einer alkoholarmen oder alkoholfreien Zubereitung, welche Kräuter oder Kräuterextrakte aus der oben genannten Gruppe umfasst.

## Beschreibung

Die Erfindung betrifft eine alkoholarme oder alkoholfreie Zubereitung, umfassend Wasser, Süßungsmittel und eine Vielzahl von Kräutern oder Kräuterextrakten. Als Kräuter oder Kräuterextrakte sollen im Folgenden alle Pflanzen oder Pflanzenteile bzw. deren Extrakte verstanden werden.

Aus dem Stand der Technik sind verschiedene Zubereitungen für Schwedenbitter bekannt. Schwedenbitter werden auch als Schwedenkräuter bezeichnet und sind seit langem im Handel erhältlich. Bei diesen Zubereitungen handelt es sich um alkoholhaltige Zubereitungen, die Kräuter oder Kräuterextrakte enthalten. Es handelt sich somit um eine Bitterspirituose, die auch als Naturheilmittel gilt. Die Anwendung kann innerlich oder äußerlich erfolgen.

Neben diesen aus dem Stand der Technik bekannten Fertigprodukten sind auch die Grundzutaten ("Schwedenkräuter") bekannt. Ebenso gehören Anleitungen zur Herstellung von Schwedenbitter zum Stand der Technik.

Wie aus dem Namen Schwedenbitter abgeleitet werden kann, wurden derartige Zubereitungen und die Erkenntnis über deren Wirksamkeit erstmals in Schweden entdeckt. Dies erfolgte Ende des 17. Jahrhunderts. Nachdem das Wissen um Schwedenbitter mindestens teilweise in Vergessenheit geraten war, wurde es Ende des 20. Jahrhunderts insbesondere durch die österreichische Kräuterkundige Maria Treben und ihren 1980 veröffentlichten Bestseller "Gesundheit aus der Apotheke Gottes" wieder in das Bewusstsein einer breiten Öffentlichkeit gebracht.

Derartige Kräuterzubereitungen kommen mit unterschiedlichsten Bezeichnungen und Zusammensetzungen in den Handel und zur Anwendung. Einem Schwedenbitter wird eine wohltuende und teils heilende Wirkung zugerechnet. Insbesondere soll durch Schwedenbitter eine Vielzahl von Missempfindungen und Leiden gelindert werden.

Eine beispielhafte Zusammensetzung von Schwedenbitter besteht aus Branntwein, Kornschnaps oder ähnlichem dem 6,7 g Aloe, Enzianwurzel oder Wermutpulver, 3,3 g Myrrhe, 0,13 g Safran. 6,7 g Sennesblätter, 6,7 g Kampfer, 6,7 g Rhabarberwurzel, 6,7 g Zittwerwurzel, 6,7 g Manna cannelata, 6,7 g Theriak venezian, 3,3 g Eberwurzwurzel und 6,7 g Angelikawurzel zugegeben werden. Oft wird Schwedenbitter auch Pfefferminze oder Pfefferminzextrakt zugesetzt.

Auch wenn die hohe Alkoholkonzentration dieser Zusammensetzung evtl, eine desinfizierende Wirkung aufweisen könnte, hat sich herausgestellt, dass er zumindest in einigen Anwendungen negative Auswirkungen hat. Insbesondere bei dauerhafter innerer Anwendung ist eine Reduzierung des Alkoholgehalts wünschenswert.

Gleichzeitig sollte die Wirksamkeit der Zubereitung in Bezug auf die wohltuende und teils heilende Wirkung jedoch nicht reduziert und bevorzugt sogar gesteigert werden.

Der Erfindung liegt daher die Aufgabe zugrunde eine alkoholarme oder alkoholfreie Zubereitung bereitzustellen, die die aus dem Stand der Technik bekannten Zubereitungen für Schwedenbitter ersetzen kann.

Außerdem liegt der Erfindung die Aufgabe zugrunde, ein Verfahren zur Herstellung einer alkoholarmen oder alkoholfreien Zubereitung bereitzustellen, die die aus dem Stand der Technik bekannten Verfahren für die Zubereitung für Schwedenbitter ersetzen kann.

Diese Aufgabe wird durch eine alkoholarme oder alkoholfreie Zubereitung, umfassend Wasser, Süßungsmittel und mindestens 12 Kräuter oder Kräuterextrakte, wobei die Kräuter oder Kräuterextrakte aus einer Gruppe ausgewählt sind, die Wermut, Eibisch, Angelika, echte Angostura, Anis, Sternansis, Beifußkraut, Orangenschale, Grüntee, Cardamon, Saflor, Gewürznelken, Tausendguldenkraut, Kamille, Zimtrinde, Koriander, Cubebenpfeffer, Attichwurzel, Zwergholunderwurzel, Fenchel, Galgantwurzel, Enzian, Alantwurzel, Artischocke, Pfefferfrüchte (schwarz), Iriswurzel, Walnußblätter, Süßholz, Süßholzsaft, Melisse, Bitterklee, Muskatblüte, Macis, Muskatnuss, Myrrha, Salbei, Bohnenkraut, Quendel, wilder Thymian, Thymian; Tormentill, Rotwurzel, Zittwerwurzel und Ingwer oder deren Extrakte umfasst.

Eine solche Zusammensetzung weist eine dem oben genannten Schwedenbitter mindestens vergleichbare wohltuende und/oder heilende Wirkung auf, ohne jedoch die Nachteile des hohen Alkoholgehalts zu haben.

Um diese Wirkung zu erreichen, ist eine gegenüber einem alkoholischen Schwedenbitter andere Zusammensetzung und Auswahl an Kräutern notwendig. Bevorzugt wird eine Auswahl von mindestens 15, besonders bevorzugt mindestens 20 Kräutern aus der oben genannten Gruppe verwendet.

Besonders bevorzugt sind Zubereitungen, die die Kräuter oder Kräuterextrakte von, mindestens 8, besonders bevorzugt mindestens 10 der Kräuter Eibisch, Angelika, Grüntee, Attichwurzel, Enzian, Iriswurzel, Walnußblättern, Süßholzsaft, Muskatnuss, Myrrha und Zittwerwurzel umfassen. Diese Kräuter bilden bevorzugt neben Wasser und Süßungsmittel die Hauptbestandteile der Zubereitung. Andere Kräuter liegen in der Zubereitung bevorzugt In Mengenanteilen < 0,5 %, bevorzugt < 0,2 %, besonders bevorzugt < 0.1 %vor.

Weiter bevorzugt werden die oben genannten Kräuter oder Kräuterextrakte in einem Masseanteil von 0,05 - 5 %, bevorzugt 0.1 - 3 % in der Zubereitung eingesetzt. Weiter bevorzugt liegt der Masseanteil von Angelika, Grüntee, Enzian, Iriswurzel, Walnußblättern, Süßholzsaft, Muskatnuss und Zittwerwurzel im Bereich zwischen 0,2 und 1,0 %.

Bevorzugt liegen die Kräuter oder Kräuterextrakte jeweils in Massenanteilen von bis zu 5 % der Zubereitung vor. Üblicherweise werden die durch die jeweiligen Kräuter hervorgerufenen Effekte jedoch schon bei weitaus geringeren Konzentrationen erreicht. Daher liegen die meisten Kräuter oder Kräuterextrakte in der Zubereitung bevorzugt in Massenanteilen von bis zu 2 Masse-% vor.

Wie oben beschrieben, ist die erfindungsgemäße Zubereitung alkoholarm oder sogar alkoholfrei. Bevorzugt weist die Zubereitung daher einen Gesamtalkoholgehalt von weniger als 2 Vol.%, bevorzugt weniger als 1 Vol.-%, weiter bevorzugt weniger als 0,5 Vol.-% auf.

In einer besonders bevorzugten Ausführungsform enthält die Zubereitung keinen Alkohol.

Durch den geringen Alkoholgehalt ist jedoch nicht ausgeschlossen, dass Kräuter oder die sonstigen Pflanzen bzw. Pflanzenteile während ihrer Aufbereitung zur Verwendung in der Zubereitung nicht mit Alkohol in Kontakt kommen können. Insbesondere eine alkoholische Extraktion der Kräuter ist hier denkbar. Zur Verringerung des Alkoholgehalts ist an ein solches Extraktionsverfahren jedoch bevorzugt ein Konzentrationsschritt angeschlossen, durch den Alkohol aus dem Extrakt zumindest weitgehend entfernt wird.

Bevorzugt ist - sofern das jeweilige Kraut oder die Pflanze bzw. der Pflanzenteil nicht unmittelbar eingesetzt (z.B. als Saft) werden kann - der jeweilige Kräuterextrakt ein durch wässrige oder alkoholische Extraktion oder Sublimation aus dem jeweiligen Kraut hergestellter Extrakt.

Sofern es sich um einen wässrigen Extrakt handelt, ist dieser bevorzugt ein wässrige Extraktion bei einer Extraktionstemperatur im Bereich von 85 - 95°C, bevorzugt im Bereich von 88 - 92°C, besonders bevorzugt von 90°C hergestellter Extrakt.

Insbesondere unpolare oder schwach polare organische Verbindungen wie z.B. ätherische Öle sind mit Wasser jedoch nicht immer in ausreichendem Maße extrahierbar. In diesem Fall bietet sich eine alkoholische Extraktion an. Wie oben beschrieben wird nach einer solchen alkoholischen Extraktion jedoch bevorzugt der Alkoholgehalt reduziert, um den Gesamtalkoholgehalt der Zubereitung gering zu halten. Bevorzugt folgt einer alkoholische Extraktion und anschließende eine Evaporation des Alkohols aus dem hergestellten Extrakt. Somit ist eine Zubereitung bevorzugt in der ein Kräuterextrakt ein durch alkoholische Extraktion und anschließende Evaporation des Alkohols hergestellter Extrakt ist.

Bevorzugt ist die Zubereitung ein Schwedenbitter.

Es konnte festgestellt werden, dass Pfefferminze Wechselwirkungen mit anderen Wirkstoffen aufweist und die Wirksamkeit einiger Medikamente reduziert. Insbesondere im Bereich der Homöopathie sind Wechselwirkungen zwischen homöopathischen Mitteln und Pfefferminze bekannt und ein viel diskutiertes Thema. Bevorzugt weist die Zubereitung daher Pfefferminze oder Pfefferminzextrakte in einem Massen-Anteil von weniger als 0,2 Massen-%, bevorzugt weniger als 0,1 Massen-% auf. Besonders bevorzugt weist die Zubereitung keine Pfefferminze oder Pfefferminzextrakte auf.

Einige der verwendeten Kräuter verleihen der Zubereitung einen bitteren Geschmack. Insbesondere für die innere Anwendung ist dies unerwünscht. Daher ist ein Süßungsmittel vorgesehen. In einer bevorzugten Ausführungsform der Zubereitung ist umfasst dieses Süßungsmittel Sorbit und/oder ein Sorbitsalz. Weiter bevorzugt handelt es sich bei dem Sorbitsalz um Kaliumsorbat.

Bevorzugt wird das Süßungsmittel in einem Massenanteil von 2 - 10 %, bevorzugt 3 - 8 %, besonders bevorzugt 4 - 5 % der Zubereitung zugesetzt.

Aufgrund des fehlenden oder reduzierten Alkoholanteils ist die Haltbarkeit einer solchen Zubereitung oft beschränkt. In einer bevorzugten Ausführungsform ist daher vorgesehen, dass die Zubereitung weiterhin ein Konservierungsmittel umfasst. Bevorzugt handelt es sich dabei um Natriumbenzoat.

Weiterhin wird die Aufgabe durch ein Verfahren zur Herstellung einer alkoholarmen oder alkoholfreien Zubereitung gelöst, welches folgende Schritte umfasst:
- Bereitstellen von Kräutern oder Kräuterextrakten welche aus einer Gruppe ausgewählt sind, die Wermut, Eibisch, Angelika, echte Angostura, Anis, Stemansis, Beifußkraut, Orangenschale, Grüntee, Cardamon, Saflor, Gewürznelken, Tausendguldenkraut, Kamille, Zimtrinde, Koriander, Cubebenpfeffer, Attichwurzel, Zwergholunderwurzel, Fenchel, Galgantwurzel, Enzian, Alantwurzel, Artischocke, Pfefferfrüchte (schwarz), Iriswurzel, Walnußblätter, Süßholz, Süßholzsaft, Melisse, Bitterklee, Muskatblüte, Macis, Muskatnuss, Myrrha, Salbei, Bohnenkraut, Quendel, wilder Thymian, Thymian, Tormentill, Rotwurzel, Zittwerwurzel und Ingwer oder deren Extrakte umfasst,
- Optional eine Herstellung von Kräuterextrakten aus Kräutern,
- Herstellen einer wässrigen Zubereitung unter Einsatz von mindestens einem Süßungsmittel sowie mindestens 12 der bereitgestellten Kräuter oder Kräuterextrakte.

In einer bevorzugten Variante des Verfahrens werden bei der Herstellung der wässrigen Zubereitung mindestens 18 der bereitgestellten Kräuter oder Kräuterextrakte verwendet. Bevorzugt wird eine Auswahl von mindestens 15, besonders bevorzugt mindestens 20 Kräutern aus der oben genannten Gruppe verwendet. Weiter bevorzugt werden bei der Herstellung der wässrigen Zubereitung mindestens 24 der bereitgestellten Kräuter oder Kräuterextrakte eingesetzt.

In einer besonders bevorzugten Variante wird der Zusatz von Pfefferminze und/oder deren Extrakte vermieden.

In einer besonders bevorzugten Variante wird der Zusatz von Alkohol(-en) vermieden oder auf ein Minimum beschränkt, dementsprechend wir der Zubereitung bevorzugt lediglich eine Alkoholmenge (evtl. in einem oder mehreren Kräuterextrakten enthalten) zugesetzt, so dass der Gesamtalkoholgehalt der Zubereitung von weniger als 2 Vol.%, bevorzugt weniger als 1 Vol.-%, weiter bevorzugt weniger als 0,5 Vol.-% beträgt.

In einer weiter bevorzugten Variante wird - sofern es nicht gewünscht wird oder es nicht möglich ist, das Kraut selbst einzusetzen - der jeweilige Kräuterextrakt durch wässrige oder alkoholische Extraktion oder durch Sublimation aus dem jeweiligen Kraut hergestellt. Dies kann besonders schonend bei vermindertem Druck erfolgen. Dadurch ist es möglich, die Extraktionstemperatur gering zu halten, was eine besonders schonende Behandlung der zu extrahierenden Substanzen gewährleistet. Insbesondere unerwünschte (teilweise) Zersetzung von zu extrahierenden Substanzen kann somit vermieden werden.

Bevorzugt ist eine Verfahrensvariante, bei der der jeweilige Kräuterextrakt durch wässrige Extraktion bei einer Extraktionstemperatur im Bereich von 85 - 95°C, bevorzugt im Bereich von 88 - 92°C, besonders bevorzugt von 90°C hergestellt wird. Diese Temperaturen sind für die meisten der zu extrahierenden Stoffe verträglich, so dass keine (oder nur eine geringe) Zersetzung erfolgt. Außerdem kann eine solche Temperatur vorteilhaft sein, um z.B. Bitterstoffe zu zersetzen. Sollte eine geringere Temperatur notwendig/bevorzugt sein, kann die Extraktion bei vermindertem Druck erfolgen.

Können die gewünschten Extrakte aus einem Kraut/aus einigen Kräutern nicht in ausreichende Menge, Qualität, Reinheit und/oder Zusammensetzung durch wässrige Extraktion gewonnen werden, wird gemäß einer bevorzugten Verfahrensvariante ein Kräuterextrakt durch alkoholische Extraktion und anschließende Evaporation des Alkohols hergestellt. Somit wird einerseits gewährleistet, dass die gewünschten Substanzen in ausreichender Menge, Qualität, Reinheit und/oder Zusammensetzung extrahiert werden können. Andererseits kann durch die der Extraktion nachgeschaltete (zumindest teilweise) Evaporation des zur Extraktion genutzten Alkohols der Alkoholanteil der Zubereitung geringe gehalten werden, oder sogar jeglicher Alkohol in der Zubereitung vermieden werden.

In einer bevorzugten Verfahrensvariante wird als Süßungsmittel Sorbit und/oder ein Sorbitsalz, bevorzug Kaliumsorbat zugesetzt. Sorbit und/oder ein Sorbitsalze, insbesondere Kaliumsorbat sind auf dem Markt in großem Mengen verfügbar, üblicherweise gut verträglich und als Süßungsmittel, insbesondere für an Diabetes leidende Personen, besonders geeignet. Bevorzugt wird das Süßungsmittel in einem Massenanteil von 2 -10 %, bevorzugt 3 - 8 %, besonders bevorzugt 4 - 5 % der Zubereitung zugesetzt.

In einer bevorzugten Verfahrensvariante wird der Zubereitung ein Konservierungsmittel, bevorzugt Natriumbenzoat zugesetzt. Natriumbenzoat ist als Konservierungsmittel besonders bevorzugt, da es für Lebensmittel zugelassen ist und üblicherweise gut verträglich ist. Der Eigengeschmack der Zubereitung wird durch den Zusatz von (dem leicht sauer, evtl. auch leicht bitter, schmeckenden) Natriumbenzoat als Konservierungsmittel nicht oder nur in geringem Maße verändert.

Die Anmelderin behält sich vor, sämtliche der in den Anmeldeunterlagen offenbarten Merkmale als erfindungswesentlich zu beanspruchen, falls diese einzeln oder in Kombination neu gegenüber dem Stand der Technik sind.

## Patentansprüche

1. Alkoholarme oder alkoholfreie Zubereitung, umfassend Wasser, Süßungsmittel und mindestens 12, bevorzugt 18, besonders bevorzugt 24 Kräuter oder Kräuterextrakte, wobei
die Kräuter oder Kräuterextrakte aus einer Gruppe ausgewählt sind, die Wermut, Eibisch, Angelika, echte Angostura, Anis, Sternansis, Beifußkraut, Orangenschale, Grüntee, Cardamon, Saflor, Gewürznelken, Tausendguldenkraut, Kamille, Zimtrinde, Koriander, Cubebenpfeffer, Attichwurzel, Zwergholunderwurzel, Fenchel, Galgantwurzel, Enzian, Alantwurzel, Artischocke, Pfefferfrüchte (schwarz), Iriswurzel, Walnußblätter, Süßholz, Süßholzsaft, Melisse, Bitterklee, Muskatblüte, Macis, Muskatnuss, Myrrha, Salbei, Bohnenkraut, Quendel, wilder Thymian, Thymian, Tormentill, Rotwurzel, Zittwerwurzel und Ingwer oder deren Extrakte umfasst.

2. Zubereitung nach Anspruch 1,
**dadurch gekennzeichnet, dass**
die Zubereitung einen Gesamtalkoholgehalt von weniger als 2 Vol.%, bevorzugt weniger als 1 Vol.%, weiter bevorzugt weniger als 0,5 Vol.-% aufweist.

3. Zubereitung nach einem der vorangegangenen Ansprüche,
**dadurch gekennzeichnet,dass**
die Zubereitung keinen Alkohol enthält.

4. Zubereitung nach einem der vorangegangenen Ansprüche,
**dadurch gekennzeichnet, dass**
der jeweilige Kräuterextrakt ein durch wässrige oder alkoholische Extraktion oder Sublimation aus dem jeweiligen Kraut hergestellter Extrakt ist.

5. Zubereitung nach Anspruch 4,
**dadurch gekennzeichnet, dass**
ein Kräuterextrakt ein durch wässrige Extraktion bei einer Extraktionstemperatur im Bereich von 85 - 95°C, bevorzugt im Bereich von 88 - 92°C, besonders bevorzugt von 90°C hergestellter Extrakt ist.

6. Zubereitung nach Anspruch 4,
**dadurch gekennzeichnet,dass**
ein Kräuterextrakt ein durch alkoholische Extraktion und anschließende Evaporation des Alkohols hergestellter Extrakt ist.

7. Zubereitung nach einem der vorangegangenen Ansprüche,
**dadurch gekennzeichnet, dass**
die Zubereitung ein Schwedenbitter ist.

8. Zubereitung nach einem der vorangegangenen Ansprüche,
**dadurch gekennzeichnet, dass**
die Zubereitung Pfefferminze oder Pfefferminzextrakte in einem Massen-Anteil von weniger als 0,2 Massen-%, bevorzugt weniger als 0,1 Massen-% aufweist und besonders bevorzugt keine Pfefferminze oder Pfefferminzextrakte aufweist.

9. Zubereitung nach einem der vorangegangenen Ansprüche,
**dadurch gekennzeichnet, dass**
das Süßungsmittel Sorbit und/oder ein Sorbitsalz, bevorzug Kaliumsorbat umfasst.

10. Zubereitung nach einem der vorangegangenen Ansprüche,
**dadurch gekennzeichnet, dass**
sie weiterhin ein Konservierungsmittel, bevorzugt Natriumbenzoat umfasst.

11. Verfahren zur Herstellung einer alkoholarmen oder alkoholfreien Zubereitung gelöst, welches folgende Schritte umfasst:
- Bereitstellen von Kräutern oder Kräuterextrakten welche aus einer Gruppe ausgewählt sind, die Wermut, Eibisch, Angelika, echte Angostura, Anis, Sternansis, Beifußkraut, Orangenschale, Grüntee, Cardamon, Saflor, Gewürznelken, Tausendguldenkraut, Kamille, Zimtrinde, Koriander, Cubebenpfeffer, Attichwurzel, Zwergholunderwurzel, Fenchel, Galgantwurzel, Enzian, Alantwurzel, Artischocke, Pfefferfrüchte (schwarz), Iriswurzel, Walnußblätter, Süßholz, Süßholzsaft, Melisse, Bitterklee, Muskatblüte, Macis, Muskatnuss, Myrrha, Salbei, Bohnenkraut, Quendel, wilder Thymian, Thymian, Tormentill, Rotwurzel, Zittwerwurzel und Ingwer oder deren Extrakte umfasst,
- Optional eine Herstellung von Kräuterextrakten aus Kräutern,
- Optional eine Entfernen oder Reduzieren des Alkoholgehalts eines oder mehrerer Kräuterextrakts/Kräuterextrakte,
- Herstellen einer wässrigen Zubereitung unter Einsatz von mindestens einem Süßungsmittel sowie mindestens 12 der bereitgestellten Kräuter oder Kräuterextrakte.

12. Verfahren nach Anspruch 11,
**dadurch gekennzeichnet, dass**
der jeweilige Kräuterextrakt durch wässrige oder alkoholische Extraktion oder Sublimation aus dem jeweiligen Kraut hergestellt wird.

13. Verfahren nach Anspruch 12,
**dadurch gekennzeichne**t, dass
ein Kräuterextrakt durch wässrige Extraktion bei einer Extraktionstemperatur im Bereich von 85 - 95°C, bevorzugt im Bereich von 88 - 92°C, besonders bevorzugt von 90°C hergestellt wird.

14. Verfahren nach Anspruch 11 oder 12,
**dadurch gekennzeichnet, dass**
ein Kräuterextrakt durch alkoholische Extraktion und anschließende Evaporation des Alkohols hergestellt wird.

15. Verfahren nach einem der Ansprüche 11 - 14,
**dadurch gekennzeichnet, dass**
als Süßungsmittel Sorbit und/oder ein Sorbitsalz, bevorzug Kaliumsorbat zugesetzt wird.
